# EUROPEAN PATENT APPLICATION

(11) **EP 4 285 839 A1**
(43) Date of publication of application: **06.12.2023**
(21) Application number: 22315115.0
(22) Date of filing: 02.06.2022
(51) Int. Cl.: A61B 17/04, A61B 17/00, A61F 5/00, A61B 17/30, A61B 17/064, A61B 17/068

(54) **APPARATUS FOR REDUCING THE SIZE OF A HOLLOW INTRACORPOREAL TISSUE STRUCTURE**

(71) Applicant: Caranx Medical SAS, 75013 Paris (FR)
(72) Inventor: BERTHET-RAYNE, Pierre, 06800 Cagnes-sur-Mer (FR); SMITS, Jonas Victor Hamen, 3360 Bierbeek (BE); SEJOR, Eric, 06000 Nice (FR)
(74) Representative: Hepp Wenger Ryffel AG

(57) **Abstract**

An apparatus (10) for reducing the size of a hollow intracorporeal tissue structure (60) comprising a housing body (11) configured for insertion within a patient having at least one compartment (13) hosting a prehensile component for attaching to at least a portion of an intracorporeal structure and optionally an affixing component.

## Description

The present invention relates an apparatus and a method of use for reducing the size of a hollow intracorporeal tissue structure according to the independent claims.

A number of operating techniques have been developed over the years to treat diseases affecting internal systems in the least invasive manner possible. The development of such instruments and techniques has enabled medical practitioners to address issues from within a patient's body and such instruments and techniques have proven to be an effective way to manage and treat certain diseases.

Gastro-intestinal tract diseases are some of the most common diseases to be treated using minimally invasive techniques. For example, obesity is known to be an increasingly preoccupying issue affecting more and more of the world's population. Classed as a chronic disease, obesity can often induce other life-threatening non-communicable diseases such as cardiovascular diseases, respiratory diseases or type-2 diabetes.

Although obesity and obesity induced non-communicable diseases may be managed with pharmaceutical medicaments and other therapies, the most efficient way to resolve the disease is to directly address the root cause which is the patient's excess weight. Many methods of treating obesity exist such as dietary changes, increased physical activity or pharmaceutical treatments. However, these methods are not always efficient enough in the long term or present disadvantages such a regain of weight once the method of treatment is stopped.

Minimally invasive instruments have been proposed to help treat obesity, for example, laparoscopic instruments designed to be inserted through multiple incision points in a patient's abdominal wall. The .stomach wall is joined along a continuous path using a handheld stapler. A laparoscopic intervention often requires multiple incision sites a patient's abdominal wall as well as multiple practitioners for manipulating the instruments.

Endoscopic devices have also been proposed to treat obesity. However, endoscopes are usually restricted in their range of movement or may present difficulties in interacting with organ tissue. They are in particular limited in their ability to connect the opposing stomach walls along long continuous paths. As such, these devices require multiple short stomach wall segments to be connected to each other. This adds complexity to the procedure, increasing procedural time and contributing to overall surgical risk.

EP 1 759 639 A1 discloses an Apparatus for endoscopically performing gastric reduction surgery in a single step: An endoscopic gastric reduction apparatus adapted for applying a series of pledgets to anterior and posterior gastric walls for the creation of a mattress stitch suture within the stomach includes an applier having a distal end and a proximal end. The applier is secured at a distal end of a support shaft shaped and dimensioned for passage down the esophagus and into the stomach. The applier includes an applier body having a suction slot shaped and dimensioned for receiving tissue therein for the application of at least one pledget housed within the suction slot for selective coupling with tissue suctioned within the suction slot.

EP 1 749 483 A2 discloses a corkscrew style anchor for gastric restriction: A gastric reduction apparatus endoscopically draws stomach walls into apposition. The apparatus includes an applicator body having a proximal end and a distal end. The applicator body also includes a suction slot shaped and dimensioned for housing a corkscrew anchor. A firing mechanism is associated with the corkscrew anchor for rotation of the corkscrew anchor in a manner causing the corkscrew anchor to penetrate and engage tissue brought adjacent the suction slot. A method for gastric reduction is achieved by introducing a gastric reduction apparatus as disclosed above within the stomach of an individual, applying the corkscrew anchor to a stomach wall and drawing stomach walls together to create a cavity within the stomach.

An endoscopic device is proposed in US 2008/262 516 A1. This patent application discloses a device for the apposition of two portions of tissue. The device disclosed provides at least two suction ports on the outer surface of a capsule, wherein the suction ports are separated by the capsule so as to physically separate the tissues captured by the multiple suction ports. The device also provides a tissue securement device for securing the separate portions of tissue captured by the separate suction ports. This device allows the securing of a succession of folds distinctly separate from one another.

With the device of US 2008/262 516 A1 it would not be possible to obtain an efficient reduction in size of an internal organ as the separate suction ports would only allow for securing of individual tissues folds.

It is an object of the present invention to overcome the drawbacks of the prior art and in particular at least one of the above mentioned disadvantages of the prior art. In particular, the device and method according to the present invention shall allow an efficient, easy to use and fast reduction in size of an internal organ.

These and other objects are solved with an apparatus and a method according to the independent claims.

The apparatus according to the invention is adapted to reduce a hollow intracorporeal tissue structure, preferably an organ, to a smaller size. The apparatus may comprise a housing body configured for insertion within a patient and the housing body may have one or more compartments that may extend at least a part of the length of the housing body. The compartment(s) may host one or more of a prehensile component, and optionally, an affixing component.

The intracorporeal tissue structure may be defined as being an organised aggregate of cells designed to work together so as to perform specific functions. A hollow tissue structure may be any structure wherein the tissue defines a cavity, for example an organ, compatible with the insertion of an apparatus for reducing the tissue structure to a smaller size.

The prehensile component may comprise one or more prehensile elements for temporarily attaching to a tissue structure and/or for repositioning one or more portions of tissue with regards to a second at least partly distanced portion of tissue. In particular, the prehensile elements are attachable to an inner wall of the tissue structure from the inside.

In some embodiments the prehensile component is configured to pull one or more portions of tissue into the housing body, optionally into one or more compartments. Further optionally, it is configured to bring two at least partly distanced and/or longitudinally extended portions of tissue at least partly closer together within a compartment of a housing body.

Bringing a plurality of portions of tissue at least partly closer within a housing body, or within a compartment of a housing body, may facilitate the affixing of the repositioned portions of tissue. It may further facilitate the affixing of a plurality of portions amongst themselves, thus reducing the size of the intracorporeal structure.

Optionally the apparatus may further comprise an affixing component configured to deliver one or more affixing elements to secure one or more repositioned portions of tissue with regards to a second at least partly distanced portion of tissue.

Additionally or alternatively, one or more affixing components and one or more prehensile components are at least partly distanced from one another within a compartment of the housing body. Optionally they are at least partly adjacent one another.

In some embodiments the one or more affixing components and the one or more prehensile components distanced within a compartment are configured to longitudinally translate so as to at least partly align and/or overlap with one another.

Accommodating a prehensile component and/or an affixing component within a housing body helps avoid the need for multiple instruments and thus helps make the intervention less invasive for the patient. Furthermore, accommodating the prehensile component and/or the affixing component within a compartment of the housing body may help avoid snagging or damage to internal tissue during the insertion of the apparatus and thus help to provide a safer apparatus with less risk of accidental injury to a patient.

According to the invention the housing body may comprise one or more apertures. The apertures may optionally be through-grooves extending through the entire thickness of a wall of the housing body. Additionally or alternatively, the apertures may extend at least a part of the length of the housing body.

This helps to avoid injuring intracorporeal tissue by keeping the operational components within the housing body until they are ready for use, thus avoiding snagging of the operational components on intracorporeal tissue during insertion. The presence of a longitudinally extending aperture helps the affixing of an extended portion, or extended portions, of tissue. It may allow for affixing a portion, or portions, of tissue in a continuous manner. The above characteristics help reduce the duration of an intervention as well as allowing for an easier procedure.

The housing body may provide at least two longitudinally extending apertures configured for attaching to two portions of at least partly distanced longitudinally extending portions of tissue.

The at least two apertures may facilitate repositioning of at least two portions of tissue. For example, two opposing apertures may allow for attaching; repositioning and/or affixing two opposing portions of a tissue, for example two opposing walls of an organ.

The axial position of one or more apertures may be aligned with the axial position of one or more compartments.

This facilitates the pulling of one or more portions of tissue within the housing body.

The prehensile component may be configured to at least partly operate, and additionally or alternatively, to extend through one or more apertures.

The affixing component may be configured to at least partly operate, and additionally or alternatively, to extend through one or more apertures.

In some embodiments, at least one prehensile component and at least one affixing component may be configured to at least partly operate, additionally or alternatively, to extend through one or more apertures.

Operating and/or extending a prehensile component and/or an affixing component through one or more apertures may facilitate the pulling of a portion of tissue into the housing body thus allowing for a more controlled intervention and may also provide a safer way of affixing a repositioned portion of tissue. It further may help avoid displacement of the, or a, portion of tissue before the placing of an affixing element.

The housing body may comprise a first configuration for insertion of at least a part of the housing body within a patient and a second configuration for operation of the housing body within a patient. In the first and the second configuration, the housing body may define different axes. At least a portion of the housing body may be configured to change from the first insertion configuration position to a second operational configuration position for attaching to one or more portions of tissue.

In the operational configuration, the axis of the apparatus may be at least partly deviated from the axis of the apparatus in the insertion configuration.

Operating the housing body in an out-of-line position with regards to the insertion configuration may allow for attaching to portions of tissue otherwise complicated to attach to or to reach.

In some embodiments at least a section of the housing body may be at least partly flexible, optionally the section for insertion within a patient, further optionally, the section configured to axially change position.

The housing body may be at least partly rotatable for changing between the operation configuration and the insertion configuration.

The temporary attaching to one or more portions of tissue and/or the changeable position or configuration of a housing body with regards to a first configuration of the apparatus may help the repositioning of a first portion of tissue with regards to a second at least partly distanced portion of tissue. It may further allow the repositioning of one or more portions of tissue that are difficult to reach.

The tissue structure to which the prehensile elements attach or the tissue potions which are secured may be at least partly longitudinally extending portions of tissue. The apparatus hence may be configured to attach or reposition one or more at least partly longitudinally extending portions of tissue or to affix one or more at least partly longitudinally extending portions of tissue.

Bringing a plurality of portions of tissue at least partly closer, optionally within a housing body, may facilitate the affixing of the repositioned portions of tissue. It may further facilitate the affixing of a plurality of portions amongst themselves, thus reducing the size of the intracorporeal structure.

The apparatus may be configured to simultaneously attach to, additionally or alternatively, to reposition and further additionally or alternatively to affix one or more portions of the at least partly longitudinally extending portions of tissue.

Additionally or alternatively to the above, the apparatus may be configured to individually attach to, additionally or alternatively, to reposition, further additionally or alternatively, to affix one or more portions of at least partly longitudinally extending portions of the tissue.

The apparatus may be configured to reposition a first portion of tissue at least partly closer to a second at least partly distanced portion of tissue. The apparatus may be further configured to affix the first portion of tissue and second portion of tissue along a longitudinally extended section.

The prehensile component may be configured to extend at least a part of the length of a housing body and may comprise one or more pressure differentiators for generating a difference in pressure. Additionally or alternatively, the prehensile component may further comprise one or more expandable elements.

The pressure differentiator may be configured to attach to one or more portions of tissue by means of negative pressure. In particular, the pressure differentiators may create a local under pressure in an area for contacting tissue, such that tissue may be sucked by a vacuum effect. Additionally or alternatively, the pressure differentiator may be configured to induce a change in configuration of one or more expandable elements, e.g. it may be a pneumatic actuator.

In some embodiments the pressure differentiator may be configured to induce a negative pressure within the housing body for pulling at least a portion of tissue into contact with at least a part of the housing body. Optionally the pressure differentiator may induce a negative pressure to pull at least a portion of tissue into the housing body, optionally into at least one compartment of the housing body.

In some embodiments the pressure differentiator is configured to induce a positive pressure. Optionally a positive pressure may induce the releasing of an attached tissue.

The use of a pressure differentiator provides an easier means for attaching to and releasing one or more portions of tissue in a non-injurious manner. It may also facilitate adapting and or changing of the portion of tissue attached, for example in the case where the wrong or an insufficient length of tissue is grasped. It may also help avoid the retraction of the apparatus if multiple reductions in an intracorporeal structure are needed.

The expandable element(s) may be configured to change between an expanded and a non-expanded state. One or more expandable elements may comprise one or more grasping tools configured to change between an inactive state and active state for attaching to one or more portions of tissue.

The change in configuration of the expandable element may be facilitated by a variation of pressure within the expandable element, optionally induced by a pressure differentiator. The change in configuration of the expandable element may induce the grasping of at least a portion of tissue.

The change in internal pressure of the expandable element may be facilitated by the pressure differentiator; wherein the pressure differentiator induces a negative and/or a positive pressure.

The expandable element and grasping tool, if provided, may facilitate localised grasping of a portion of tissue or grasping of extended portions of a tissue thus helping the practitioner reduce the size of an intracorporeal structure. This feature may also help a practitioner to adapt an intervention as necessary without retracting the apparatus from within a patient.

The one or more grasping tools may be configured to change between the inactive state and the active state when the expandable element changes in configuration. Optionally the expanded state of the expandable element may correspond to the inactive state of the grasping tool and the non-expanded state of the expandable element may correspond to the active state of the grasping tool.

The change in configuration of a grasping tool and of an expandable element may facilitate the attaching to one or more portions of tissue. It may further facilitate the repositioning of a portion of tissue by retracting the grasped portion or portions of tissue into the housing body for repositioning.

The change in configuration of the expandable element may induce a retraction of the active grasping tool for pulling one or more portions of tissue, optionally the retraction of the active grasping tool pulls one or more portions of tissue into the housing body or a compartment.

The affixing component may be configured to longitudinally translate within one or more compartments. The affixing component may comprise one or more perforating elements for generating perforations in the tissue. Affixing elements may be applied in these perforations. Affixing elements might be formed by the perforating elements, e.g. by staples. Additionally or alternatively the affixing component may comprise one or more separated affixing elements for securing a repositioned tissue, e.g. sutures which can be applied in previously applied perforations.

The translation of the affixing component within the housing body allows for securing the attached portions of tissue within the housing body, this may allow for a more controlled delivery of an affixing element. It may further allow the affixing of extended portions of tissue without repositioning the apparatus. The affixing component being accommodated within a housing body may also help avoid accidental perforating of an intracorporeal structure by the perforating element.

In some embodiments one or more perforating elements may be loaded into the apparatus prior to an intervention on a patient. Optionally one or more perforating elements are loaded into the apparatus during an intervention.

Loading one or more perforating elements prior to an intervention may help reduce the duration of the intervention, thus being less strenuous for the patient and the practitioner. The possibility of loading the perforating element during an intervention may help for repeating a repositioning and affixing of tissue without retracting the apparatus from within the patient thus also reducing the duration of an intervention and helping reduce stress on the patient and practitioner.

In some embodiments the perforating element may be configured to advance longitudinally within the housing body, optionally a compartment of the housing body. The longitudinal advancement is operated by means of a control unit situated at or near a proximal end of the apparatus. Optionally, the perforating element may be further configured to advance in a rectilinear manner or a helical manner.

The affixing component may deliver one or more affixing elements individually or simultaneously to one or more portions of tissue.

Delivering one or more affixing element individually may facilitate more selective intervention where only localised portions of tissue are to be affixed. A simultaneous delivery of affixing elements allows for affixing portions of tissue in one step and thus may reduce the duration of an intervention.

The affixing element may be at least partly malleable for changing conformation and may adopt the general conformation performed by previously applied perforations.

The use of a malleable material for an affixing element may help to avoid tension of the affixing element securing one or more portions of tissue. This may help to avoid detaching and/or breakage of the affixing element under contractile movement of a tissue, for example the stomach.

In some embodiments one or more affixing elements may be loaded into the apparatus prior to an intervention on a patient, additionally or alternatively, one or more affixing elements may be loaded into the apparatus during an intervention.

Loading one or more affixing elements prior to an intervention may help reduce the duration of the intervention, thus being less strenuous for the patient and the practitioner. The possibility of loading the affixing element during an intervention may help for repeating a repositioning and affixing of tissue without retracting the apparatus from within the patient thus also reducing the duration of an intervention and helping reduce stress on the patient and practitioner.

The affixing component may further comprise a guide extending at least a part of the length of one or more compartments. The guide may be configured to assist the longitudinal translation of a perforating element. Additionally or alternatively, the guide may assist the longitudinal translation of an affixing element.

The guide may comprise one or more grooves configured to interact with a perforating element or with an affixing element.

The at least one groove may be configured to assist the placing of the affixing element. Optionally, the guide comprises a plurality of grooves for guiding one or more perforating elements and/or affixing elements. One or more grooves may extend at least a part of the length of the guide. Optionally one or more grooves are at least partly extending in a circumferential direction. Further optionally, at least one groove of the guide may have a circular shape on the surface of the affixing component. It may have an at least partly circular cross-section.

The guide may facilitate the advancement of a perforating element thus helping the delivery of the affixing element. This in turn may help to maintain the portion(s) of tissue in a repositioned state before placing of the affixing element(s). The, or a, plurality of grooves of the guide, assist in guiding the advancement of the perforating element,so as to place the affixing. element. The grove, or grooves, may also assist in the securing of an affixing element thus helping the affixing of a repositioned tissue.

The invention may provide a system comprising the apparatus and a control unit. The system may further have at least one, optionally a plurality of actuators. The actuator(s) may be comprised in and/or controlled by the control unit. The actuator(s) may be configured to convert an input, optionally originating from the control unit, so as to operate one or more parts and/or elements of the apparatus.

The actuators may be any of electrical (for example electromagnetic; electrohydraulic; piezoelectric); magnetic; mechanical; fluid (pneumatic and/or hydraulic); thermal. Furthermore, the actuators may be configured to operate remotely and/or automatically.

Additionally or alternatively at least one, optionally at least some, of the actuator(s) may be positioned within/along the apparatus. It/they may be positioned in a portion of the apparatus that may be inserted within the patient. Optionally it/they may be positioned, or may also be positioned, in a portion of the apparatus that is not designed to be inserted within the patient. Additionally or alternatively, at least one actuator may be positioned in the control unit of the system.

In a second aspect the invention may provide a method of reducing a hollow intracorporeal tissue structure to a smaller size. The method may optionally use an apparatus according the first aspect of the invention. The method may further comprise one or more steps of:
(i) attaching a prehensile component to a hollow intracorporeal tissue structure, preferably an organ, especially from the inside of the organ; and/or
(ii) pulling on one or more portions of tissue to reposition at least a portion of tissue; and/or
(iii) securing the repositioned tissue using a affixing component.

### Brief Description of the Drawings

The invention will be better understood with reference to the following decryption of preferred embodiments and the accompanying drawings, which show:
Fig. 1a-b are schematic perspective views of an apparatus for reducing the size of an intracorporeal structure.
Fig. 2a-b are schematic longitudinal cross-sections of an apparatus for reducing the size of an intracorporeal structure in an insertion (Fig. 2a) and in an operation configuration (Fig. 2b).
Fig. 3a-b are schematic cross-sections of an alternative embodiment of an apparatus for reducing the size of an intracorporeal structure in a plan perpendicular to the axis of the apparatus in an insertion (Fig. 3a) and in an operation configuration (Fig. 3b).
Fig. 4a is a schematic perspective view of an alternative embodiment of an apparatus for reducing the size of an intracorporeal structure.
Fig. 4b-c are schematic longitudinal sections of the apparatus of Fig. 4a for reducing the size of an intracorporeal structure.
Fig. 5 is a schematic longitudinal cross-section of an alternative embodiment of an apparatus for reducing the size of an intracorporeal structure.
Fig. 6 is a schematic cross-section of an affixing component of an apparatus for reducing the size of an intracorporeal structure in an operation configuration.
Fig. 7 is a schematic cross-section of an affixing component of an apparatus for reducing the size of an intracorporeal structure in a operation configuration
Fig. 8 is a schematic longitudinal cross-section of an alternative embodiment of an apparatus for reducing the size of an intracorporeal structure.
Fig. 9 is a schematic longitudinal cross-section of an alternative embodiment of an apparatus for reducing the size of an intracorporeal structure.
Fig. 10 is a schematic longitudinal cross-section of an alternative embodiment of an apparatus for reducing the size of an intracorporeal structure.
Fig. 11 is a schematic longitudinal cross-section of an alternative embodiment of an apparatus for reducing the size of an intracorporeal structure.
Figs. 12a-j are schematic diagrams illustrating steps in an example technique for reducing a hollow intracorporeal tissue structure to a smaller size.

### Detailed Description of Some Embodiments:

Non-limiting embodiments of the invention are now described by way of example only.

In the illustrations, the same reference numerals are used to denote the same or equivalent features amongst different embodiments and examples. Unless described to the contrary, the description of a feature in one embodiment may also apply to the same or equivalent feature in one embodiment or example. Features may also be interchanged in embodiments as desired.

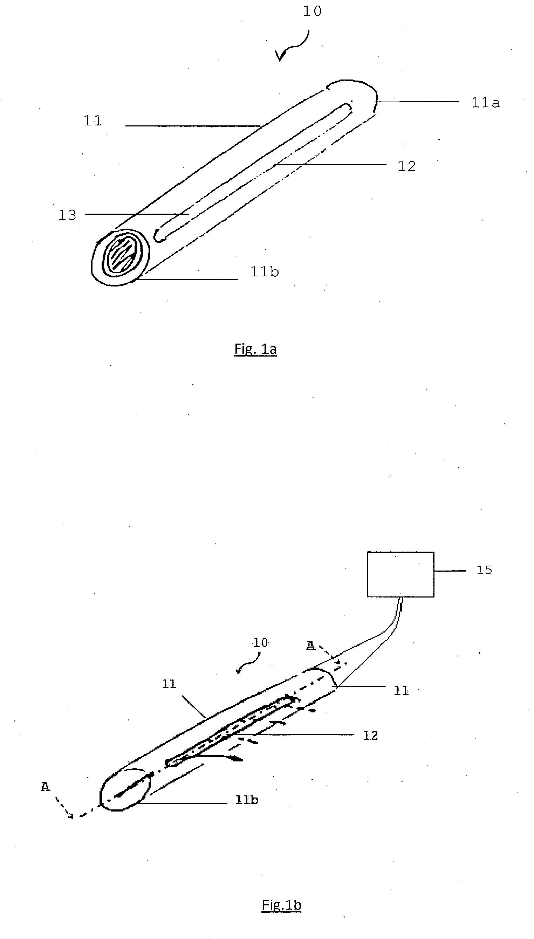

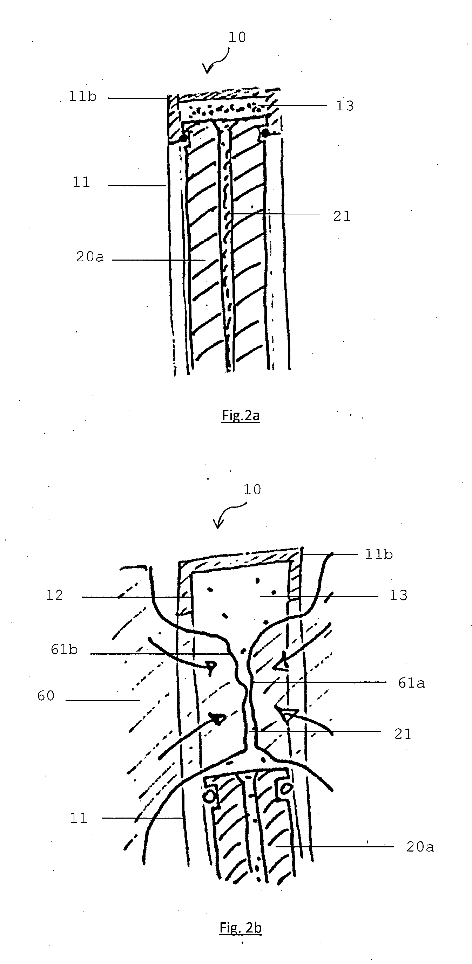

Referring to figures 1a and 1b, an apparatus 10 configured for reducing the size of a hollow intracorporeal tissue structure 60 (see Fig. 2b) of a patient is shown. The hollow intracorporeal tissue structure 60 may preferably be an organ, for example, an organ of the digestive system such as the stomach.

The apparatus 10 comprises a housing body 11 at least partly configured for insertion within a patient and further comprises a prehensile component 20 and an affixing component 30, as will be shown in Fig. 2 and 3 and 5 to 7, respectively.

The apparatus 10 is configured for at least partial insertion within a patient, such as for trans-oral insertion. The housing body 11 extends from a proximal end 11a toward a distal end 11b. At least a section of the distal end 11b is configured for insertion within a patient.

Materials and dimensions can be chosen largely in line with conventional endoscopes for gastric sleeve surgery or the Apollo OverStitch^{™} Endoscopic Suturing System.

At least part of the apparatus may be made of any biocompatible material suitable for insertion within a patient.

At least part of the apparatus 10 may be made from an at least partly flexible material. Additionally or alternatively, the apparatus or a part thereof, may be made of any medical grade metal. For example, stainless steel and/or nitinol may be used to make at least a part of the apparatus.

Additionally or alternatively, at least a part of the apparatus may be made of any suitable polymer. Polymers that may be used are, for example, thermoplastics and/or fluoropolymers.

Exemplary thermoplastics that may be used for the outer cover of the housing are polyether ether ketone (PEEK); polyethylene terephthalate (PET); thermoplastic polyurethane (TPU); polyurethane (PUR).

Exemplary fluoropolymers that may be used for inner parts of the housing are polytetrafluoroethylene (PTFE); fluorinated ethylene-propylene (FEP); perfluoroalkoxy alkane (PFA).

The housing body may have a length ranging between 75-90mm.

The diameter of the housing body may be constant along at least a part of the length of the housing body. Additionally or alternatively, the diameter of the housing body may at least partly vary along the length of the housing body. The housing body may have an outer diameter ranging between the following: 5mm-40mm; 5mm-30mm; 5mm-20mm; 5mm-10mm; 10mm-20mm.

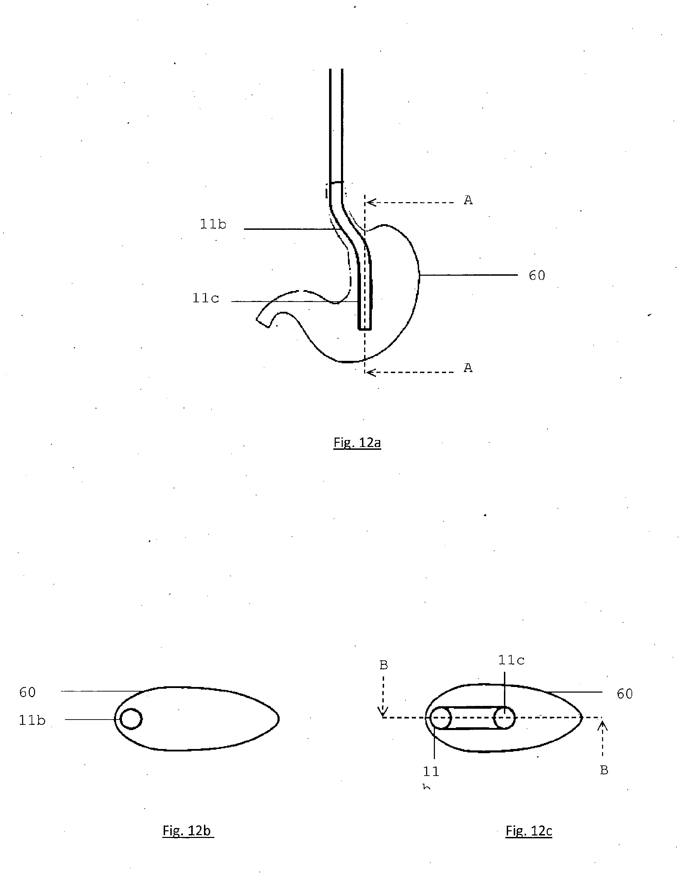

As will be shown with reference to Figs. 12a-i, the apparatus 10 is configured for reducing a hollow intracorporeal-tissue structure 60 to a smaller size 60'. For this purpose, the apparatus 10 is configured to reduce the size of an intracorporeal tissue structure 60 by repositioning one or more portions of tissue 61.

As shown in Figs. 2a and 2b, the apparatus 10 is further configured to temporarily attach to one or more portions of an intracorporeal tissue structure 61a, 61b for repositioning. The apparatus 10 is configured to reposition a first portion of tissue 61a with regards to a second at least partly distanced portion of tissue 61b. The apparatus 10 is configured to reposition a first portion of tissue 61a at least partly closer to a second portion of tissue 61band in particular to reposition a first portion of tissue 61a and a second portion of tissue 61b in contact with one another.

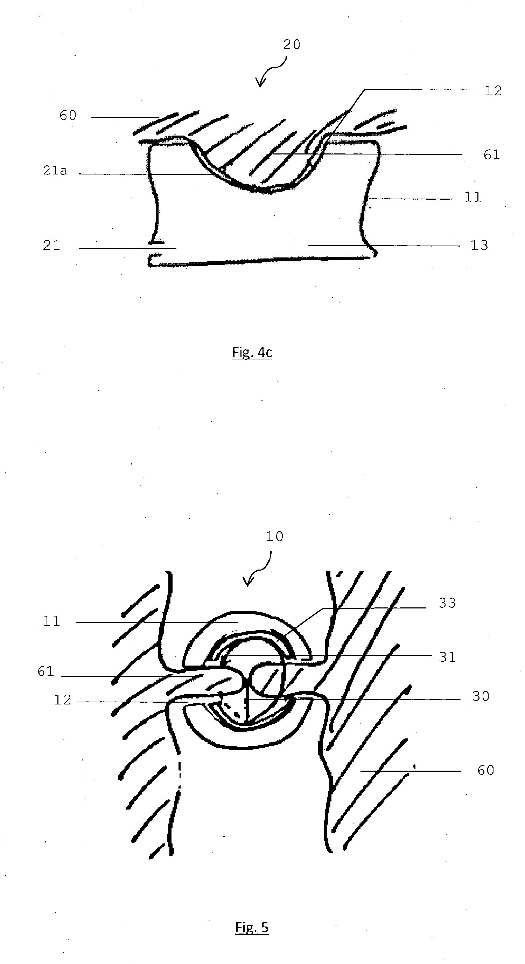

As will be explained in Fig. 5 to 7, the apparatus 10 is configured to affix one or more portions of tissue 61c. The one or more portions- of tissue 61 may be, for example, internal portions of an organ wall, optionally opposing walls of an organ.

The apparatus 10 is configured to affix one or more portions of tissue 61c in an at least partly longitudinal manner, optionally along one or more portions of tissue extending at least partly longitudinally.

The apparatus 10 is configured to attach to one or more longitudinally elongated portions of tissue and to reposition one or more longitudinally elongated portions of tissue and to affix one or more longitudinally extended portions of tissue in a longitudinally extending manner.

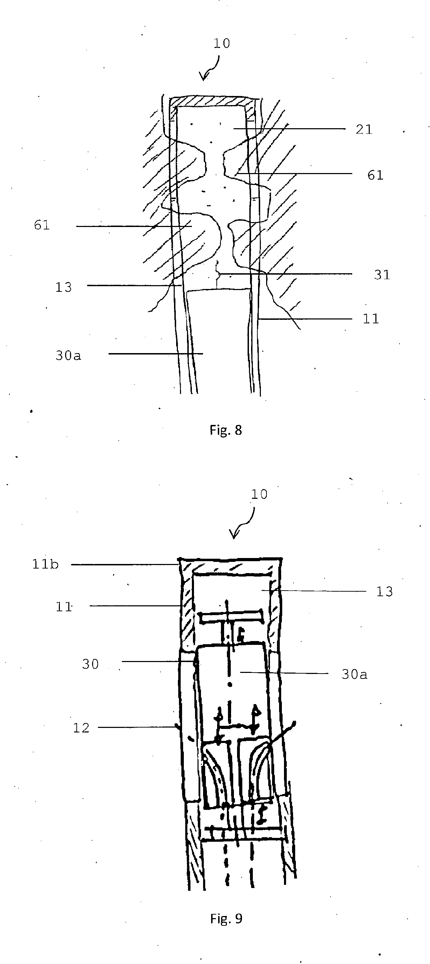

Additionally or alternatively, as will be seen in figs. 8 to 11 the apparatus 10 may be configured to attach to and/or reposition one or more smaller portions of tissue and/or one or more extended portions of tissue. It may be further configured to attach to and/or reposition one or more smaller portions of tissue and/or one or more expanded portions of tissue simultaneously and/or individually.

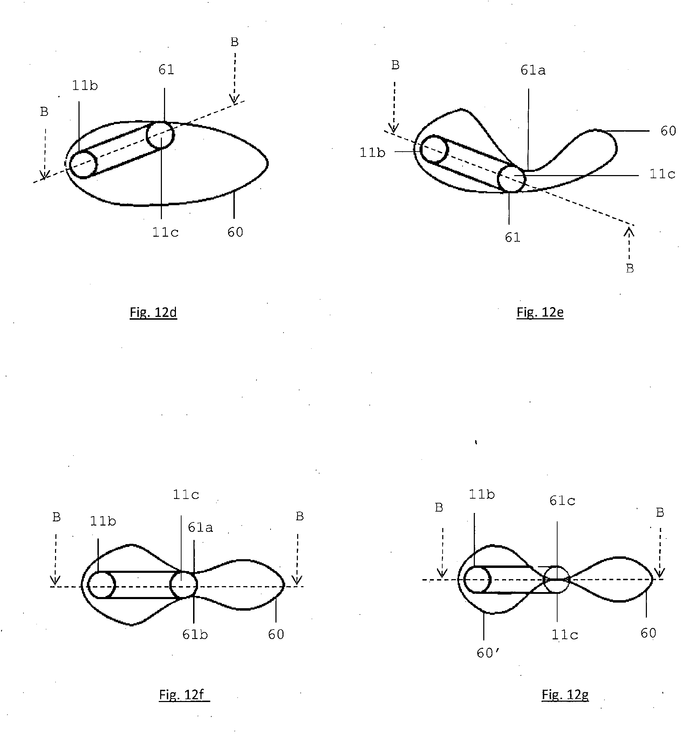

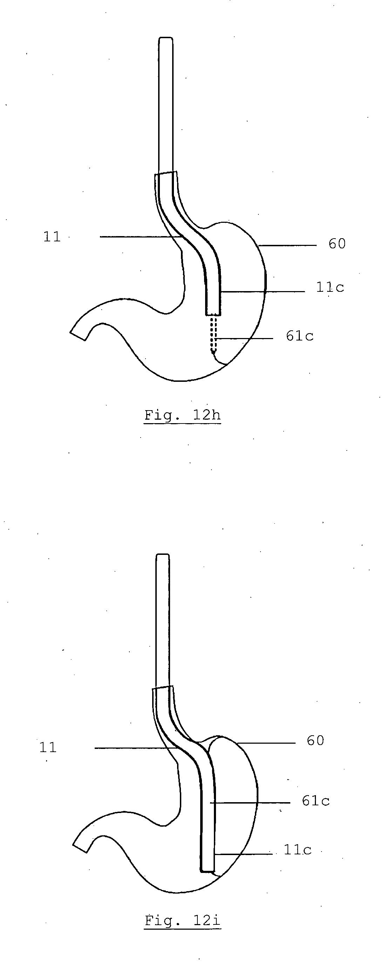

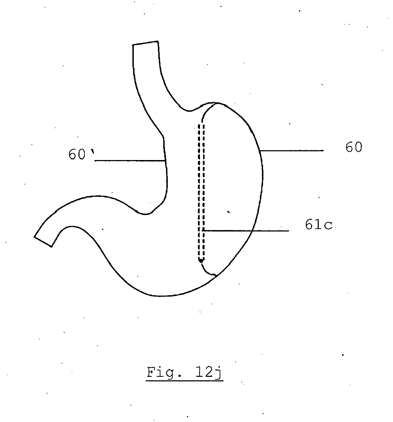

As will be shown in Fig. 12, the apparatus 10 may be configured to have an at least partly adaptable axial configuration. The apparatus 10 may have one or more axial configurations, for example, a first configuration for insertion within a patient and a second configuration for operating the apparatus. In the insertion configuration, the apparatus 10 has an axis A (see Fig. 1b). In the second operational configuration, the apparatus has an axis B, different from the axis a (see Fig. 12). The insertion axis A may facilitate insertion within a patient trough a natural opening in a patient. The operational axis B is configured to at least partly deviate with regards to the insertion axis A for attaching and/or repositioning one or more portions of tissue 61.

The adaptable portion of the apparatus is provided by a flexible configuration of the housing body, optionally a longitudinal portion of the housing body 11c, for example a portion neighbouring a distal end 11b of the housing body 11.

In other words, a section of the housing body 11c (see fig. 12a) may be configured to at least partly deviate to an out-of-line position with regards to an 'insertion axis A for attaching to one or more portions of tissue.

The adaptable portion of the apparatus may have'a length ranging between 5-25mm.

Optionally, the adaptable portion may be bendable and deviate to an angle ranging between 0°-60° from the insertion axis A. Optionally, the adaptable portion may deviate to any angle ranging between the following: 0°-10°; 0°-20°; 0°-30°; 0°-40°; 0°-50°.

The adaptable portion of the housing body may be made from an at least partly flexible material for deviating from the insertion axis.

Additionally or alternatively the adaptable portion of the housing body may be made from an at least partly rigid material.

Optionally, the adaptable portion may have one or more cut-outs along its length. The one or more cut-outs may be longitudinal and/or at least partly circumferential.

In other words, the adaptable portion of the housing device may be made from an at least partly rigid material and may comprise one or more cut-out in said material to allow an at least partial flexibility of the adaptable portion. For example, the adaptable portion may be made of metal, such as stainless steel and/or nitinol provided with cut-outs.

The cut-outs may confer an at least partial flexibility to the ' housing body and/or to a portion of the housing body. This may facilitate an at least partial change in angle of the adaptable portion of the housing body.

The axial deviation may facilitate the attaching of the apparatus to portions of tissue otherwise difficult to access. It may further provide a better range of motion during the intervention, and help avoid any retracting of the apparatus for repositioning of the device, and thus may reduce the duration of the intervention.

The apparatus 10 may further provide a control unit 15 (fig. 1b) situated at or near the proximal end 11a of the housing body 11. Operation of the control unit 15 may induce one or more or all of: moving a section of the housing body 11c out-of-line with regards to the insertion axis A of the apparatus 10; rotation of a section of the housing body 11c with regards to the axis A or the axis B.

As shown in Fig. 2a, the housing body 11 comprises a compartment 13 that extends at least part of the length of the housing body 11 and which houses a prehensile component 20. Optionally, the housing body 11 may comprise a plurality of compartments 13 that may extend at least part of the length of the housing body 11.

The compartment 13, optionally the plurality of compartments 13, may host one or more prehensile components 20.

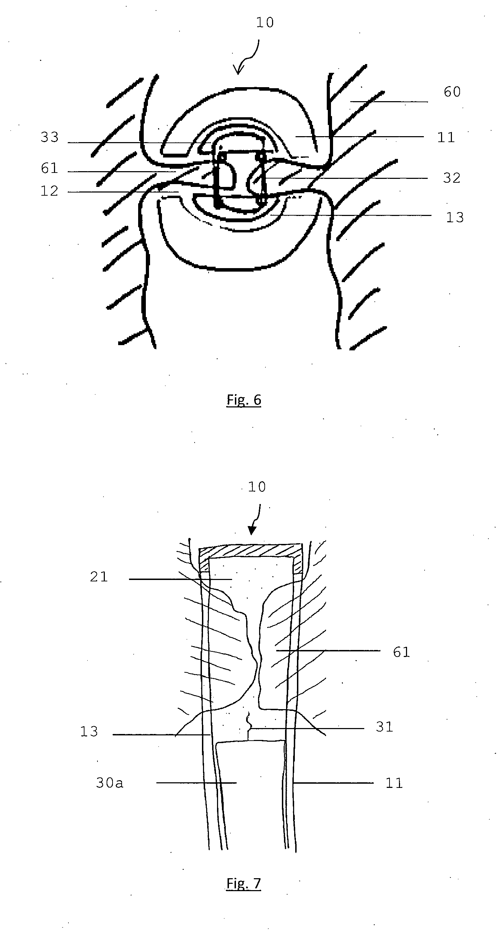

The housing body 11 comprises one or more apertures 12 that extend at least a part of the length of the housing body 11 and are configured to allow contact between at least a portion of intracorporeal tissue 61 and one or more prehensile components 20 (see Fig. 2b) and one or more affixing components 30 (see Fig. 6 and 7).

In some embodiments the housing body 11 may be sealed at a distal end 11b.

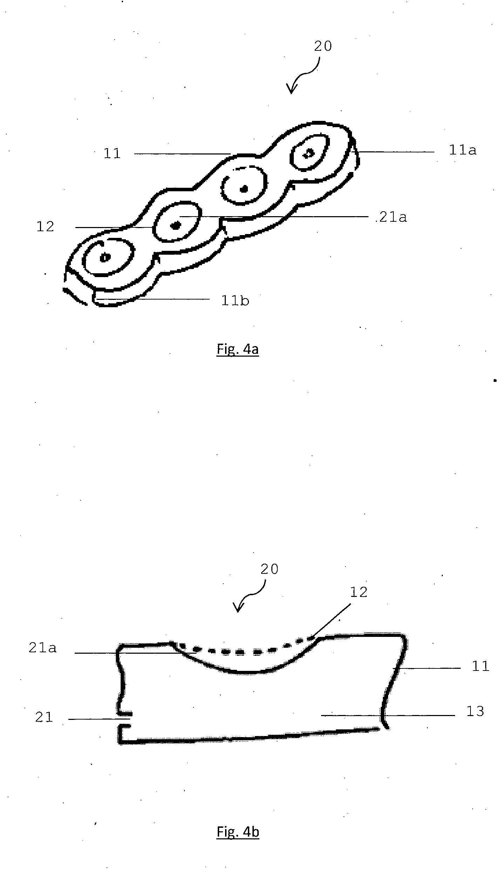

The axial position of the aperture 12 is at least partly axially aligned with the axial position of one or more compartments 13. The aperture 12 of the housing body 11 is formed as a through-groove. The through-groove may longitudinally extend at least a part of the length of the housing body 11. Optionally, at least one groove may have an at least partly circular shape (Fig. 4a) or and/or an at least partially circular cross-section on the surface of the housing body (see fig. 4b).

The aperture may facilitate a single continuous affixing of a portion or a plurality of portions of tissue, thus helping to reduce the size of an intracorporeal tissue structure.

The aperture further allows the prehensile component and/or the affixing component to be accommodated by the housing body whilst still being able to interact with intracorporeal tissue.

The one or more apertures allow one or more prehensile components and/or affixing components to operate through the one or more apertures.

The aperture 12, optionally a plurality of apertures 12, may facilitate bringing distanced portions of tissue at least partly closer to one another; for example, it may help bring opposite sides of a tissue structure, for example an organ, closer, in particular within a compartment of the housing body 11.

In some embodiments, as shown in Figs. 8, the housing body 11, optionally a section of the housing body 11, may be configured to at least partially rotate around an axis A. The housing body 11, optionally a section of the housing body 11c, may be at least partly malleable to change from an aligned and rather straight position with regards to an insertion axis A (see Fig. 1b) to an at least partly out-of-line curved position with regards to the insertion axis A of the apparatus 10 (see Figs. 8).

The position of at least a section of the housing body 11c with regards to an axis A may be adapted by means of the control unit 15 situated at or near a proximal end 11a of the housing body 11 (see fig. 1b).

In other words the housing body 11 may be configured to be deformable to at least partially deviate from an insertion axis A for attaching to portions of tissue that are complicated to attach to with a device that remains straight.

Figs. 2-4 show a prehensile component 20 configured for attaching at least temporarily to one or more portions of intracorporeal tissue 61.

Optionally, the prehensile component 20 is further configured for repositioning a first portion of tissue 61a with respect of a second portion of tissue 61b, wherein the first portion of tissue 61a and second portion of tissue 61b are at least partly distanced from one another and further wherein the repositioning of the first portion of tissue 61a and/or the second portion of tissue 61b reduces at least partly the distance between the first portion of tissue 61a and second portion of tissue 61b.

The prehensile component 20 extends at least a part of the length of a housing body 11; optionally extending from a proximal end 11a toward a distal end 11b of the housing body 11. The prehensile component 20 comprises a shaft 20a configured to longitudinally translate within a housing body 11, optionally configured to longitudinally translate within one or more compartments 13 of a housing body 11.

The prehensile component 20 is configured to operate within one or more compartments 13 of the housing body 11. It is further configured to operate through one or more apertures 12 (see fig. 1a-b), optionally through one or more through-grooves extending the length of the housing body 11. Optionally, the prehensile component 20 is configured to operate through one or more longitudinally extending apertures 12 of the housing body 11.

The shaft 20a translates at least a part of the length of a housing body 11, optionally of the length of one or more compartments 13 of a housing body 11.

In some embodiments the prehensile component 20 is configured to reposition one or more portions of tissue 61. It is also configured to reposition a first portion of tissue 61a (see fig. 2b and 12) with regards to a second portion of tissue 61b, wherein the second portion of tissue 61b is at least partly distanced from the first portion of tissue 61a.

The prehensile component 20 is operable by means of the control unit 15 (see fig. 1b) situated at or near a proximal end 11a of the housing body 11. Operation of the control unit 15 induces one or more of or all of: longitudinal translation of a shaft 20a; activation of a prehensile component; longitudinal translation of a prehensile component, attaching of a prehensile component to one or more portions of tissue 61; repositioning of one or more portions of tissue 61.

In some embodiments the prehensile component 20 comprises a prehensile element configured to temporarily attach to a portion 61 of an intracorporeal tissue structure 60, for example, a portion of organ tissue. Optionally, the prehensile component 20 comprises a plurality of prehensile elements for attaching to a plurality of portions of tissue 61. The prehensile element, optionally the plurality of prehensile elements, is accommodated by at least one compartment 13 of the housing body 11.

Additionally or alternatively, the prehensile component 20 is configured to attach to one or more portions of tissue 61, optionally longitudinally extending portions of tissue, simultaneously and/or individually. Optionally, it is further configured for repositioning one or more portions of tissues 61 simultaneously and/or individually.

Bringing a plurality of portions of tissue at least partly closer within a housing body 11, or within a compartment 13 of a housing body 11, may facilitate the affixing of the repositioned portions of tissue 61. It may further facilitate the affixing of a plurality of portions amongst themselves, thus reducing the size of the intracorporeal structure.

Additionally or alternatively, one or more prehensile elements are configured to extend at least a part of the length of the housing body 11. It, or they, operate at least partly through one or more apertures 12 of the housing body 11. optionally, one or more prehensile elements extend at least partly through at least one aperture 12 of the housing body 11.

In the embodiment illustrated in figs. 2a-b and 4a-c the prehensile element is configured to operate within a housing body 11. The prehensile element comprises a pressure differentiator 21 configured to create a pressure differential for attaching to a portion of tissue, optionally for attaching to a plurality of portions of tissue.

In the embodiment of fig. 4, the prehensile component 20 provides a suction component 21a, optionally a plurality of suction components 21a, for transmitting a negative pressure.

The suction component 21a may be any means suitable for transmitting a negative pressure, for example, a suction cup. At least one suction component 21a may be situated within the housing body 11 of the apparatus 10.

The suction component 21a, optionally a plurality of suction components 21a are situated at and/or near one or more apertures 12 of a housing body 11 and are configured to attach to at least a portion of tissue 61 by means of negative pressure. The suction component 21a, optionally a plurality of suction components 21a, axially align with one or more at least partly circular apertures 12.

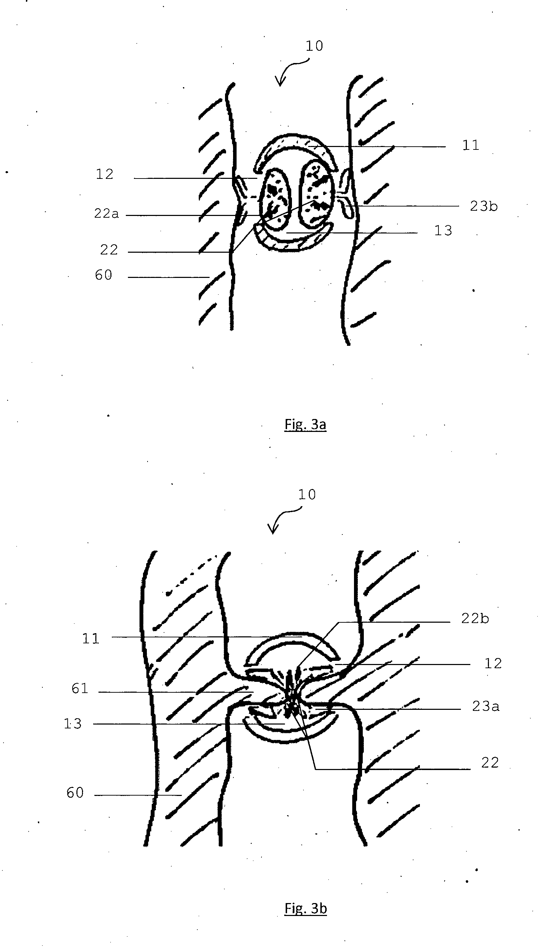

Referring to fig. 3a-b, the prehensile component 20 accommodated by a compartment 13 of a housing body 11 comprises an expandable element 22 for attaching to one or more portions of tissue and configured to change from an expanded state 22a to a non-expanded state 22b.

The change in configuration of the expandable element 22 is facilitated by a variation of pressure within the expandable element 22, optionally induced by a pressure differentiator. The change in configuration of the expandable element 22 induces the grasping of at least a portion of tissue 61.

The change in internal pressure of the expandable element 22 is facilitated by the pressure differentiator 21. The pressure differentiator 21 induces a negative pressure and/or a positive pressure.

The pressure differentiator may create a pressure differential, for example, by use of hydraulic pressure or pneumatic pressure.

The expandable element 22 may be any material and/or means suitable for changing from the expanded state 22a to the non-expanded state 22b or for changing from the non-expanded 22b state to the expanded state 22a. The expandable element 22 may be for example a balloon.

In some embodiments, the expandable element 22 comprises one or more grasping tools 23 for attaching to one or more portions of tissue 61. The grasping tool 23 may be configured to change from' an inactive state 23b to an active state 23a. The grasping tool 23 is configured for attaching to or grasping at least a portion of tissue 61 in an active state 23a.

In some embodiments the change in configuration of an expandable element 22 induces a change in configuration of a grasping tool 23 from an inactive state 23b to an active state 23a.

The grasping tool 23 may be any suitable means for at least temporarily attaching to and/or pulling one or more portions of intracorporeal tissue e.g. the grasping tool may be a pair of clamps.

Additionally or alternatively, the grasping tool 23 is configured to pull at least a portion of tissue 61 into contact with at least a part of the housing body 11. Optionally, the grasping tool 23 is configured to pull at least a portion of tissue 61 into the housing body 11, optionally into a compartment 13 of the housing body 11.

Referring to figs. 2-11, in some preferred embodiments the housing body 11 provides two, optionally more, apertures 12extending at least a part of the length of the housing body 11 and are generally adjacent one another. Optionally the apertures 12 are at least partly parallel to one another.

The at least two apertures may facilitate repositioning of at least two portions of tissue. For example, two opposing apertures may allow for attaching; repositioning and/or affixing two opposing portions of a tissue, for example two opposing walls of an organ.

The elements composing the prehensile component 20 are operable by the control unit 15 situated at or near a proximal end 11a of the housing body 11 (see fig. 1b). Operation of the control unit 15 induces one or more of or all of: generating a negative pressure; generating a positive pressure; changing the internal pressure of an expandable element; attaching to a portion of tissue; attaching a grasping tool to a portion of tissue; pulling a portion of tissue into contact with the housing body 11; pulling a portion of tissue 61 into the housing body 11 and/or into at least one compartment 13 of the housing body 11; repositioning of a portion of tissue; releasing a portion of tissue.

Referring to figs. 5-11, in some embodiments the apparatus 10 provides an affixing component 30 for delivering an affixing element 32 to a repositioned portion of tissue 61.

The affixing component 30 extends at least a portion of the length of the housing body 11 and comprises a shaft 30a housed by the housing body 11; optionally housed by at least one compartment 13. The affixing component further comprises one or more perforating elements 31 and/or one or more affixing elements 32.

In some embodiments the affixing component 30 is further configured to longitudinally translate within the housing body 11; optionally it longitudinally translates from a proximal end 11a toward a distal end 11b of the housing body 11, for delivering one or more affixing elements 32 to a repositioned portion,' or repositioned portions, of tissue 61.

Additionally or alternatively, the affixing component 30, optionally the shaft 30a, is configured to longitudinally advance within the housing body 11 in an at least partly rectilinear manner; optionally it progresses in a helical manner.

The affixing component 30 progressively advances at least a part of the length of the housing body 11, or of a compartment 13, optionally advancing from a proximal end 11a toward a distal end 11b of the housing body 11. The motion of the affixing component 30 is rectilinear within the housing body 11 and/or it is a helical motion.

In some embodiments the longitudinal translation of the affixing component 30 induces a longitudinal motion of the perforating element 31 and/or the affixing element 32.

Additionally or alternatively in some embodiments, the perforating element 31 and/or the affixing element 32 advance in a rectilinear manner, optionally a helical manner, within at least a part of the housing body 11, optionally within a compartment 13 of the housing body 11.

The perforating element 31 and/or affixing element 32 are configured to advance simultaneously with one another and/or with the affixing component 30.

In some embodiments the affixing component 30 is configured to operate within a housing body 11, optionally within at least one compartment 13 of the housing body 11. Additionally or alternatively, the affixing component 30 is configured to extend and/or operate through at least one aperture 12 of the housing body 11.

The affixing component 30 is operated by means of the control unit 15 situated at or near a proximal end 11a of the apparatus 10.

Operation of the control unit 15 induces one or more of or all of: a longitudinal translation of the affixing component 30 and/or a shaft 30a; a longitudinal advancement of a perforating element 31 and/or of the affixing element 32; a rectilinear and/or a helical motion of the perforating element 31 and/or of the affixing element 32.

The affixing component 30 comprises one or more perforating elements 31 configured to perforate one or more portions of tissue 61. The perforating element 31 is configured to perforate a portion of tissue 61 so as to place an affixing element 32 to secure the portion of tissue 61.

In other words the perforating element may facilitate the delivering of an affixing element and may perforate at least one portion of tissue so as to deliver an affixing element. Optionally, the perforating element may facilitate the delivery of a plurality of affixing elements.

As shown in figs. 6, 7 and 8, the perforating element 31 has an at least partly spiral configuration and is configured to deliver an affixing element 32.

In some embodiments, the affixing element 32 has an initial at least partly linear conformation and is configured to change to an at least partly spiral conformation.

The affixing element 32 is at least partly malleable for changing conformation and for securing one or more repositioned portions of tissue.

It is configured to adopt the general conformation of a perforating element 31 or to adopt the general conformation formed by previously performed perforations.

The affixing element may be made of any suitable at least partly malleable material for changing configuration. It may be made of a biocompatible material. It may be, for example, a suture and/or an at least partly malleable thread-like element.

A shown in figs. 9, 10 and 11, the shaft of the affixing component 30a is configured to longitudinally translate within the housing body 11 for attaching to one or more portions of tissue 61. Optionally, the perforating element 31 of the affixing component extends at least partly through an aperture 12 of the housing body 11 for attaching to the one or more portions of tissue and further configured to deliver one or more affixing elements 32. Additionally or alternatively the perforating element 31a is configured to deliver one or more anchor elements 32b for securing the one or more affixing elements 32 to the tissue portion 61. The one or more anchor elements 32b are configured to secure one or more affixing elements, for example a suture, with regards to a portion of tissue.

The perforating element 31 may be any means suitable for perforating a portion of tissue 61, for example a needle 31a.

In some embodiments one or more perforating elements 31 are loaded into the apparatus 10 prior to an intervention on a patient. Optionally one or more perforating elements 31 are loaded into the apparatus 10 during an intervention.

Optionally the perforating element 31 is connected to a shaft 30a of the affixing component 30, additionally or alternatively, connected to an affixing element 32.

In some embodiments the perforating element 31 is configured to advance longitudinally within the housing body 11, optionally a compartment 13 of the housing body 11. The longitudinal advancement is operated by means of the control unit 15 situated at or near a proximal end 11a of the apparatus 10. Optionally, the perforating element 31 is further configured to advance in a rectilinear manner and/or a helical manner.

Additionally or alternatively, the longitudinal advancement of the perforating element 31 is driven by the longitudinal advancement of the shaft 30a. Optionally, the perforating element 31 and the shaft 30a advance in unison or simultaneously.

In some embodiments the affixing component'30 is further configured for securing one or more repositioned portions of tissue. Optionally, it is configured for affixing a first repositioned portion of tissue 61a with regards to a second portion of tissue 61b.

Additionally or alternatively, the affixing component 30 is configured to deliver one or more affixing elements 32 along a longitudinally elongated section to secure one or more longitudinally elongated portions of tissue.

In other words the affixing component 30 may be configured to deliver an affixing element 32 for securing one or more portions of tissue 61. The affixing element 32 may secure a first portion of tissue 61a with regards to a second portion of tissue 61b.

Additionally or alternatively, the affixing component 30 provides a plurality of affixing elements 32 for securing a portion of tissue 61, optionally for securing a plurality of portions of tissue 61. The plurality of affixing elements 32 are placed simultaneously and/or successively.

The affixing component 30 is configured to deliver one or more affixing elements 32 to one or more portions of tissue 61, optionally to one or more longitudinally extending portions of tissue.

In some embodiments the affixing component 30 is further configured to simultaneously deliver one or more affixing elements 32 to one or more portions of tissue and/or to one or more longitudinally extending portions of tissue.

Additionally or alternatively, the affixing component 30 is further configured to individually deliver one or more affixing elements 32 to one or more portions of tissue 61 and/or to one or more elongated portions of tissue.

In some embodiments the affixing component 30 is configured to deliver one or more affixing elements 32 along a longitudinally extended section, optionally along one or more longitudinally extending portions of tissue.

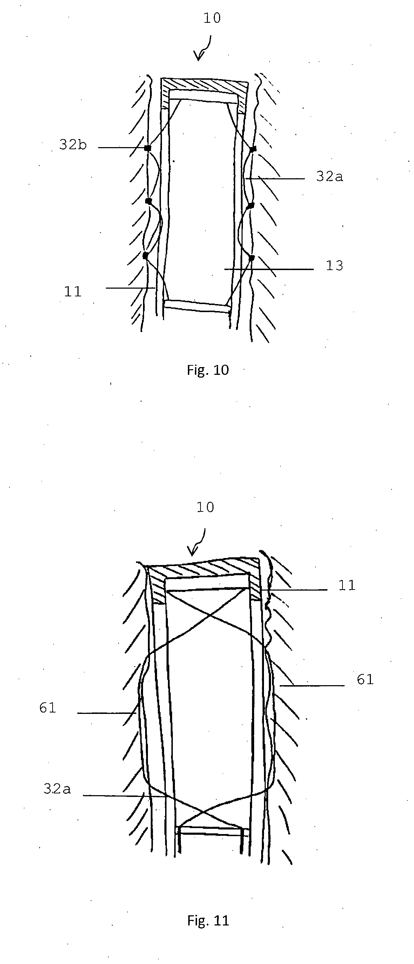

As shown in fig. 10, in some embodiments the affixing component 30 is configured to deliver one or more anchor elements 32b for securing an affixing element 32.

The affixing element 32 provides a thread-like element 32a for securing a portion of tissue 61. Optionally the thread-like element 32a is configured to be used with a perforating element 31 of the affixing component 30. Further optionally, the affixing element 32 is configured to interact with an anchor element 32b.

The thread-like element may be any suitable means and/or material, for example a suture or a wire. The thread like element may interact with at least- one perforating means of the affixing component 30. Optionally the thread-like element interacts with an anchor element 32b for securing the thread-like element with regards to a portion of tissue 61.

In other words a perforating element 31 may be configured to introduce a thread-like element 32a for securing a portion of repositioned tissue 61. For example, a needle 31a may be provided for placing at least one suture to secure a portion of tissue 61. Optionally an anchor element 32b may be provided to anchor the suture to the surface of a portion of tissue.

Referring to fig. 6, the affixing element 32, optionally a plurality of affixing elements 32, is configured to perforate and/or traverse at least a portion of tissue 61. The affixing element 32 is staple-like. The staple-like element 32b may be configured to interact with the guide 33 of the affixing component 30, optionally with at least one groove 33a of the guide 33.

The elements composing an affixing component 30 are operated by means of the control unit 15 situated at or near a proximal end 11a of the apparatus 10. Operation of the control unit 15 induces one or more of or all of: a longitudinal translation of the shaft 30a; a longitudinal advancement of a perforating element 31 and/or of the affixing element 32; a rectilinear and/or a helical motion of the perforating element 31 and/or of the affixing element 32; perforating of a portion of tissue 61; perforating a plurality of portions of tissue 61; delivery of one or more affixing elements 32; affixing of at least one portion of tissue.

In some embodiments the affixing component 30 comprises a guide 33 configured to help guide one or more perforating elements 31 and/or at least one affixing element 32 of the affixing component 30. The guide 33 extends at least a part of the length of the housing body 11, optionally a part of the length of one or more compartments 13.

In some embodiments the guide 33 comprises at least one groove 33a for guiding the longitudinal motion of a perforating element 31. The at least one groove 33a is configured to assist the placing of the affixing element 32. Optionally, the guide 33 comprises a plurality of grooves 33a for guiding one or more perforating elements 31 and/or affixing element 32. One or more grooves 33a extend at least a part of the length of the guide 33. Optionally one or more grooves are at least partly circumferential. Further optionally, at least one groove 33a of the guide 33 is at least partly circular.

Referring to figs. 7 and 8, in some embodiments one or more affixing components 30 and one or more prehensile components 20 are hosted by a compartment 13 of the housing body 11. Optionally they may be hosted within the same compartment 13 of the housing body 11.

Additionally or alternatively, one or more affixing components 30 and one or more prehensile components 20 are at least partly distanced from one another within a compartment of the housing body 11. Optionally they are at least partly adjacent one another.

In some embodiments the one or more affixing components 30 and the one or more prehensile components 20 distanced within a compartment 13 are configured to longitudinally translate so as to at least partly align with one another.

Illustrated in figs. 10 and 11, in some embodiments the affixing element 32 provides a thread-like element 32a for securing a portion of tissue 61. Optionally the thread-like element. 32a is configured to be used with a perforating element 31 of the affixing component 30. Further optionally, the affixing element 32 is configured to interact with an anchor element 32b.

The thread-like element 32a may be any suitable means and/or material, for example a suture or a wire. The thread-like element 32a interacts with at least one perforating means of the affixing component 30. Optionally, the thread-like element interacts with an anchor element 32b for securing the thread-like element with regards to a portion of tissue 61.

Optionally, the thread-like element extends at least a part of the length of the housing body and is configured to bring a first portion of tissue and a second portion of tissue at least partly closer to one another.

In other words, a perforating element 31 is configured to introduce a thread-like element 32a for securing a portion of repositioned tissue 61. For example, a needle 31a is provided for placing at least one suture to secure a portion of tissue 61. Optionally an anchor element 32b is provided to anchor the suture to the surface of a portion of tissue.

A method of operation of the apparatus 10 as described above comprises a first step (i). for operating a prehensile component 20 to attach to one or more portions of tissue 61 for example an organ wall 60, a second step (ii) for pulling one or more portions of tissue 61 into the housing body 11, and a third step for operating an affixing component 30 to deliver an affixing element 32 to the repositioned portion of tissue.

Figs. 12a-g illustrate a method of reducing a hollow intracorporeal tissue structure. The drawings illustrate the repositioning of portions of a stomach wall, but the same principals can be used for other hollow intracorporeal tissue structures.

Referring to figs. 12a-b, a distal end 11b of a housing body 11 is inserted through a digestive tract and guided into the organ 60, for example the stomach, according to the insertion axis A of the housing body 11.

Illustrated in fig. 12c, at least a section of the housing body 11c inserted into the organ 60 is axially deviated according to an operational axis B so as that the section of the housing body 11c is near a first target portion of tissue 61a.

Referring to figs. 12d-f, the axially deviated section of the housing body 11c is rotated around the axis A (see fig. 12d) so that it contacts and can attach to a first portion of tissue 61a. The position of the section of the housing body 11c is then adjusted, e.g. by back rotation, to bring the first portion of tissue 61a closer to a second portion of tissue 61b. The section of the housing body 11c contacts and attaches to the second portion 61b (see fig. 12e) and is repositioned centrally with regards to the organ 60, thus repositioning the first portion of tissue 61a and second portions of tissue 61b centrally with regards to an organ 60 (see figs. 2b; 3b; 4c; 6; 7; 8-11).

Illustrated in fig. 12g, the first portion of tissue 61a and second portion of tissue 61b are affixed together 61c, e.g. after the use of an affixing component 30, along an affixed section 61c, thus reducing the organ to a smaller size 60'.

Shown in fig. 12h, in some embodiments the apparatus is configured to in a step-by-step manner progressively attach to; reposition and affix one or more portions of tissue whilst adjusting the position of the housing body. Additionally or alternatively they are affixed along a section extending at least partly the length of the organ.

Illustrated in fig. 12i, the apparatus is configured to attach to; reposition and affix one or more portions of tissue in a one-time sequential manner whilst the position of the housing body is maintained. Optionally, they are affixed along a section extending at least partly the length of the organ.

Depicted in fig.12j is an intracorporeal tissue structure 60 having been reduced in size 60' according to the method and apparatus described above and wherein two portions of tissue are affixed to one another along a longitudinally extending section of tissue 61c.

It will be appreciated that the foregoing description is merely illustrative of example forms of the invention, and that many modifications and equivalents can be used within the principles of the invention.

## Claims

1. Apparatus (10) for reducing the size of a hollow intracorporeal tissue structure, preferably an organ, wherein the apparatus comprises a housing body (11) for insertion within a patient and wherein at least one compartment (13) extends within at least a part of the length of the housing body (11), optionally extending from or near a proximal end (11a) toward a distal end (11b), and wherein one or more of said at least one compartment (13) house:
(i) a prehensile component (20) having one or more prehensile elements (21) for temporarily attaching to a tissue structure and/or for repositioning one or more portions of tissue (61) with regards to a second at least partly distanced portion of tissue, and optionally,
(ii) an affixing component (30) for delivering one or more affixing elements (31) to secure one or more repositioned portions of tissue (61) with regards to a second at least partly distanced portion of tissue.'

2. Apparatus according to claim 1, wherein the housing body (11) comprises one or more apertures (12), optionally wherein one or more apertures (12) are through-grooves and/or extend along at least a part of the length of the housing body (11).

3. Apparatus according to claim 2, wherein the axial position of one or more apertures (12) aligns with the axial position of one or more compartments (13).

4. Apparatus according to any claim 2 or 3, wherein a prehensile component (20) and/or the affixing component (30) are configured to operate and/or extend through one or more apertures (12) .

5. Apparatus according to any claim 1 to 4, wherein the housing body (11) comprises a first insertion configuration for insertion of at least a part of the housing body within a patient and a second operational configuration for operation of the housing body within a patient, and wherein at least a part of the housing body (11) is configured to change from the first insertion configuration to the second operational axis position, the axis of the device in the operational configuration being at least partly deviated from the axis of the device in the insertion configuration.

6. Apparatus according to claim 5, wherein the housing body (11) is at least partly rotatable for changing between the operation configuration and the insertion configuration.

7. Apparatus according to any claim 1 to 6, wherein the apparatus (10) is configured to attach to and/or reposition and/or affix one or more at least partly longitudinally extending portions of tissue (61).

8. Apparatus according to claim 7 wherein the apparatus (10) is configured to attach to and/or reposition and/or affix one or more at least partly longitudinally extending portions of the tissue (61) simultaneously and/or individually.

9. Apparatus according to any claim 8, wherein the apparatus (10) is configured to reposition a first portion of tissue (61a) at least partly closer to a second at least partly distanced portion of tissue (61b) and is further configured to affix the first portion of tissue and second portion of tissue along a longitudinally extended section (61c).

10. Apparatus according to any claim 1 to 9, wherein the prehensile (20) component extends within at least a part of the length of a housing body (11) and comprises one or more pressure differentiators (21) for generating a difference in pressure and/or wherein the prehensile component (20) further comprises one or more expandable elements (22).

11. Apparatus according to claim 10, wherein the pressure differentiator (21) is configured to attach to one or more portions of tissue (61) by means of negative pressure and/or is configured to induce a change in configuration of one or more expandable elements (22).

12. Apparatus according to claims 10 or 11, wherein the one or more expandable elements (22) are configured to change configuration between an expanded (22a) and a non-expanded state (22b) and comprises one or more grasping tools (23) configured to change between an active state (23a) and an inactive state (23b) for attaching to one or more portions of tissue (61) .

13. Apparatus according to claim 12, wherein one or more grasping tools (23) are configured to change between the inactive state (23b) and the active state (23a) when the expandable element changes in configuration, optionally wherein the expanded state (22a) of the expandable element (22) corresponds to the inactive state (23b) of the grasping tool and the non-expanded state (22b)of the expandable element (22) corresponds to the active state of the grasping tool (23a).

14. Apparatus according to any claim 1 to 13, wherein the affixing component (30) is configured to longitudinally translate within one or more compartments (13) and comprises one or more perforating elements (31) and/or one or more affixing elements (32) for securing a repositioned tissue (61).

15. Apparatus according to claim 14, wherein the affixing component (30) comprises a guide (33) extending at least a part of the length of one or more compartments (13) and configured to assist the longitudinal translation of a perforating element (31) and/or of an affixing component (32).

16. Apparatus according to claims 14 or 15, wherein the guide (33) comprises one or more grooves configured to interact with a perforating element (31) and/or an affixing element (32).

17. System comprising an apparatus according to any of the claims 1-15 and a control unit, wherein the system further comprises at least one actuator comprised in and/or controlled by the control unit.

18. Method of reducing a hollow intracorporeal tissue structure to a smaller size, the method optionally using an apparatus (10) according to any claims 1 to 16 or a system according to claim 17, the method further comprising one or more of:
(i) attaching a prehensile component (20) to a hollow intracorporeal tissue structure (60), preferably an organ; and/or
(ii) pulling on one or more portions of tissue (61) to reposition at least one portion of tissue; and/or
(iii) securing the repositioned tissue (61) using an affixing component (30).
